# EUROPÄISCHE PATENTSCHRIFT

(11) **EP 2 502 646 B1**
(45) Veröffentlichungstag und Bekanntmachung des Hinweises auf die Patenterteilung: **12.03.2014**
(21) Anmeldenummer: 12159241.4
(22) Anmeldetag: 13.03.2012
(51) Int. Cl.: A61N 1/37, A61N 1/372

(54) **Implantierbares Gerät**
Implantable device
Appareil implantable

(30) Priorität: 23.03.2011 US 201161466486 P
(43) Veröffentlichungstag der Anmeldung: 26.09.2012
(73) Patentinhaber: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Lindner-Vogt, Karin L.

(56) Entgegenhaltungen:
- EP-A1- 2 198 914
- WO-A1-98/33554
- WO-A1-2007/013917
- US-A1- 2009 210 025
- US-A1- 2010 106 215
- US-B1- 7 369 898

## Beschreibung

Die Erfindung betrifft ein Implantierbares elektromedizinisches Gerät mit einer Steuereinheit, die mit einer Diagnose- oder Therapieeinheit einer Testeinheit verbunden ist. Außerdem ist die Steuereinheit mit einer MRT-Erkennungseinheit zum Erfassen MRT-typischer magnetischer und/oder elektromagnetischer Felder und/oder mechanischer Schwingungen verbunden. Die MRT-Erkennungseinheit kann hierzu einen MRT-Sensor aufweisen.

Die Testeinheit ist ausgebildet, die Diagnose-Therapieeinheit oder Komponenten der Diagnose-Therapieeinheit zu testen und so gewonnene Testergebnisse zum Speichern (an eine Speichereinheit) auszugeben. Die Diagnose- oder Therapieeinheit hat Komponenten wie Sensoreinheiten und/oder Therapieabgabeeinheiten und ist ausgebildet, physiologische Parameter aufzunehmen und/oder eine Therapieabgabe zu bewirken. Die Steuereinheit ist ausgebildet, die Testeinheit zum Testen der Diagnose-Therapieeinheit anzusteuern.

Derartige implantierbaren medizinischen Geräte sind beispielsweise implantierbare Defibrillatoren (ICDs) oder Herzschrittmacher, die über Elektrodenleitungen elektrische Stimulationsimpulse an Herzgewebe (Myokard) abgeben können oder über entsprechende Sensoren elektrische Potentiale im Herzgewebe erfassen können. Andere Implantate wie z.B. Neurostimulatoren dienen der Stimulation anderen Gewebes. Im Zusammenhang mit Herzschrittmachern ist es bekannt, dass diese selbsttätig automatische Nachsorgen z.B. als sog. Chronjobs, d.h. hinsichtlich des Ablauf und des Zeitpunkts vorprogrammierte Selbsttests durchführen, bei denen wie nach heutigen Richtlinien üblich, bestimmte Parameter so z.B. Reizschwelle, Elektrodenimpedanz, Batteriespannung, Signalamplituden etc. erfasst werden. Hierzu dient die von der Steuereinheit angesteuerte Testeinheit.

Implantierbare Herzschrittmacher oder Defibrillatoren sind typischerweise mit Elektrodenleitungen für die Elektrostimulation verbunden, die den Nachteil mit sich bringen, dass sich ihr elektrischer Leiter in einem Kernspintomografen (auch als Magnetresonanztomografen bezeichnet) erwärmen kann, weil die im Kernspintomografen herrschenden wechselnden Magnetfelder in dem elektrischen Leiter nicht unbeachtliche elektrische Ströme induzieren. Auch können solche induzierten Ströme über Elektrodenpole der Elektrodenleitung an umliegendes Gewebe abgegeben werden und so beispielsweise zu unerwünschten Gewebeerwärmungen führen. Auch könnten induzierte Ströme Komponenten des Gerätes beeinträchtigen oder zerstören oder es könnte sich elektrischer Widerstand zwischen Potential erfassenden oder Impuls abgebenden Elektrodenpolen an der Elektrodenleitung verändern, z.B. erhöhen. Dies würde die Erfassung oder Auswertung elektrischer Signale eines Herzens und damit die Therapiesteuerung oder auch die Therapieabgabe beinträchtigen. Deshalb können Herzschrittmacherpatienten heutzutage in der Regel nicht oder nur eingeschränkt in einem Kernspintomografen untersucht werden.

An implantierbaren Herzschrittmachern oder Defibrillatoren (im Folgenden auch gemeinsam als Herzstimulatoren oder IPG (implantable pulse generator) bezeichnet) sind nämlich typischerweise wenigstens an solch eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden.

Die US2009/0210025A1 beschreibt ein Implantat, das basierend auf gemessenen Impedanzen eine dynamische Anpassung von Strömen und Spannungen vornimmt. Aus der WO2007/013917 ist ein Implantat bekannt, dass basierend auf der Erkennung von eingekoppelten Signalen eine Impedanz dynamisch verändert.

Der Erfindung liegt die Aufgabe zugrunde, eine sichere MRT-Untersuchung von Patienten mit elektronischen Implantaten zu realisieren.

Erfindungsgemäß wird diese Aufgabe durch ein implantierbares elektromedizinisches Gerät mit
- einer MRT-Erkennungseinheit zum Erfassen MRT-typischer Magnet- und/oder elektromagnetischer Wechselfelder und/oder mechanischer Schwingungen
- einer Steuereinheit, die mit der MRT-Erkennungseinheit verbunden ist
- einer Diagnose- oder Therapieeinheit und mit
- einer Testeinheit
gelöst. Die Testeinheit ist ausgebildet, die Diagnose- oder Therapieeinheit oder Komponenten der Diagnose- oder Therapieeinheit zu testen und so gewonnene Testergebnisse zum Speichern z.B. an eine Speichereinheit auszugeben. Die Diagnose- oder Therapieeinheit (im Folgenden kurz: Diagnose-Therapieeinheit) weist Sensoreinheiten und/oder Therapieabgabeeinheiten als Komponenten auf und ist ausgebildet, physiologische Parameter aufzunehmen und/oder eine Therapieabgabe zu bewirken. Die Steuereinheit ist ausgebildet, die Testeinheit zum Testen der Diagnose-Therapieeinheit anzusteuern. Erfindungsgemäß ist die Steuereinheit mit der MRT-Erkennungseinheit verbunden und ausgebildet, auf ein MRT-typisches Magnet- und/oder elektromagnetisches Wechselfeld und/oder mechanische Schwingungen anzeigendes Ausgangssignal der MRT-Erkennungseinheit hin einen Vor-Test wenigstens einer Komponente der Diagnose-Therapieeinheit durch die Testeinheit auszulösen, wobei die Testeinheit während des Vor-Tests Werte von Systemintegritäts-Parametem erfasst, die eine Betriebfähigkeit des implantierbaren medizinischen Gerätes charakterisieren. Außerdem ist das implantierbare elektromedizinische Gerät ein Stimulator oder ein Monitoring Implantat mit einem gewebekontaktierenden Elektrodenpol oder einem Anschluss für einen einem gewebekontaktierenden Elektrodenpol, ist der oder einer der Systemintegritäts-Parameter vorzugsweise ein einen Elektroden-Gewebe-Kontakt charakterisierender Impedanzwerte.

Wie eingangs erwähnt, kann die MRT-Erkennungseinheit einen MRT-Sensor aufweisen, wie er. z.B. aus US 12/970,290 bekannt ist. Die Diagnose-Therapieeinheit kann eine reine Diagnoseeinheit sein oder eine reine Therapieeinheit, z.B. eine Stimulationseinheit eines Herzschrittmachers oder eine Kombination aus beidem sein. Die Diagnoseeinheit kann beispielsweise eine aus Herzschrittmachern bekannte Sensingeinheit zum Erfassen und ggf. Auswerten von Herzereignissen anzeigenden elektrischen Potentialen sein.

Das erfindungsgemäße implantierbare medizinische Gerät ist in der Lage, selbsttätig MRT-typische (und ähnliche) Magnet_und/oder elektromagnetische Wechselfelder und/oder mechanische Schwingungen zu erkennen und daraufhin Werte wichtiger, die eine Betriebfähigkeit des implantierbaren medizinischen Gerätes charakterisierender Systemintegritäts-Parameter zu erfassen und schafft damit die Voraussetzung, eine Veränderung dieser Werte infolge der Einwirkung solcher Felder auf das Gerät erkennen zu können.

Bislang war die MRT-Untersuchung bei Patienten mit elektronischen Implantaten wie z.B. Herzschrittmachern, ICDs, CRT-Ds oder Neurostimulatoren deswegen kontraindiziert, weil in der Umgebung des MRT Probleme wie die Erwärmung der Elektrodenspitze durch die starken elektromagnetischen Wechselfelder im MRT auftreten können. Insbesondere die Erwärmung der Elektrodenspitze kann zu einem temporären oder permanenten Reizschwellenanstieg, Unter- oder Oversensing oder gar einer Dislokation führen. Letzteres kann das erfindungsgemäße Gerät selbsttätig erfassen und erlaubt somit beispielsweise die Überprüfung eines Implantat- und Elektrodensystem nach stattgefundener MRT-Untersuchung, um Systemfehler des elektronischen Implantates und MRT-induzierte E-lektrodenprobleme zu erkennen.

Für eine nach einer MRT-Prozedur vorgeschriebene Nachsorgeuntersuchung ist bislang immer ein Implantatspezialist (hier Kardiologe/Elektrophysiologe) nötig, der die Untersuchung durchführt. Das MRT wird aber typischerweise von Radiologen durchgeführt, die nicht ausgebildet und befugt sind, die Implantatfunktionen eines elektronischen Implantates zu überprüfen. Daher muss der Patient typischerweise schon vor, vor allem aber nach der MRT-Untersuchung einen zweiten Facharzt aufsuchen. Dies erhöht die Prozedurkosten für das MRT erheblich und birgt das Risiko, dass die nachträgliche Untersuchung nicht stattfindet. Das erfindungsgemäße Gerät macht eine derartige Voruntersuchung durch einen Facharzt vor einer MRT-Prozedur überflüssig.

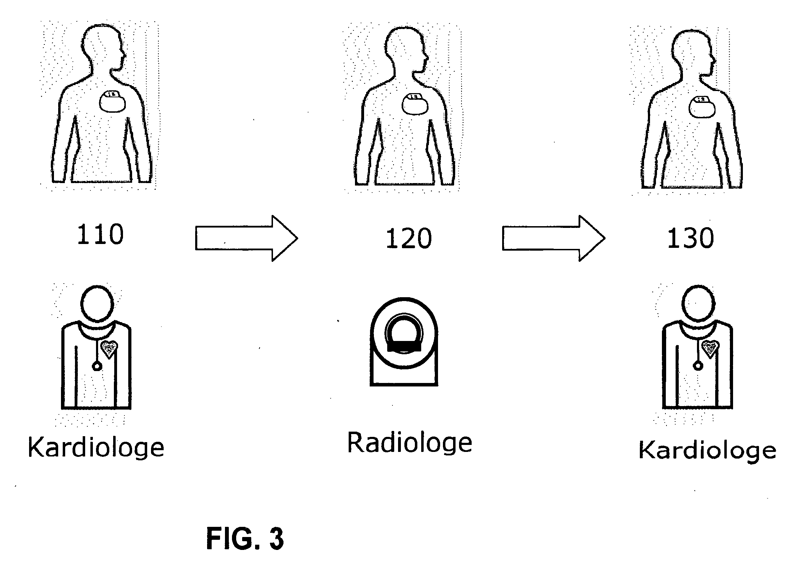

Figur 3 zum besseren Verständnis den derzeit vorgeschriebenen Ablauf einer Vor- und Nachuntersuchung eins implantierbaren medizinischen Gerätes vor und Nach einer MRT-Prozedur. Ein Patient wird unmittelbar vor einer geplanten MRT-Prozedur von einem Implantatspezialisten untersucht (110). Bei dieser Voruntersuchung (110) wird die Systemintegrität und Elektrodenmesswerte als Referenz erfasst, um diese später mit den Ergebnissen der Nachuntersuchung vergleichen zu können. U. U. wird zu diesem Zeitpunkt das Implantat auch in einem sog. MRT-sicheren Mode programmiert.

Anschließend erfolgt die eigentliche MRT-Prozedur (120) beim Radiologen.

Im unmittelbaren Anschluss an die MRT-Untersuchung muss nach den geltenden Vorschriften der Implantatspezialist eine Nachsorge (130) des Implantatsystems durchführen und die Systemintegrität u.a. durch Vergleich mit den initial ermittelten Werten bewerten.

Die Gesamtprozedur schließt gegenüber einer "normalen" MRT-Prozedur zwei zusätzliche Prozeduren bei einem Implantatspezialisten ein und setzt voraus, dass Implantatspezialist und Radiologe zeitlich und räumlich eng beieinander arbeiten, was in der Praxis nur mit deutlich erhöhten Aufwand realisierbar ist.

Vorzugsweise ist das implantierbare elektromedizinische Gerät ausgebildet, einen Wegfall eines ein MRT-typisches Magnet- und/oder elektromagnetisches Wechselfeld und/oder mechanische Schwingungen anzeigendes Ausgangssignal der MRT-Erkennungseinheit zu erfassen und daraufhin einen Nach-Test vorzugsweise derjenigen Komponente der Diagnose-Therapieeinheit durch die Testeinheit auszulösen, zu der ein Vor-Test durchgeführt wurde und bei dem vorzugsweise Werte derjenigen Systemintegritäts-Parameter erfasst werden, zu denen Werte aus einem Vor-Test vorliegen. Ein solches implantierbares elektromedizinisches Gerät macht auch eine lokale Nachuntersuchung durch beispielsweise einen Kardiologen überflüssig öder erleichtert diese zumindest. Seitens des implantierbaren elektromedizinischen Gerätes während des Vor- und des Nach-Testes erfasste Werte können nämlich telemetrisch an ein zentrales Service-Center übertragen und dort automatisch oder durch einen Arzt ausgewertet werden.

Vorzugsweise ist die Steuereinheit ausgebildet, das implantierbare medizinische Gerät in Anschluss an einen Nach-Test in einen Post-MRT-Betriebsmodus zu versetzen, in dem zyklisch Nach-Tests erfolgen, wobei die Steuereinheit weiterhin dazu ausgebildet ist, Werte eines jeweiligen Systemintegritäts-Parameters aus einem jeweiligen Nach-Test mit einem entsprechenden Wert desselben Systemintegritäts-Parameters aus den letzten Vor-Test zu vergleichen. Auf diese weise kann das implantierbare medizinische Gerät möglicherweise kritische Änderungen von werten der Systemintegritätsparamter selbsttätig erkenn.

Darüber hinaus ist die Steuereinheit vorzugsweise ausgebildet, den Post-MRT-Betriebsmodus zu beenden, wenn eine Abweichung der Werte unterhalb eines vorgegeben Schwellwertes liegt.

Falls das implantierbare elektromedizinische Gerät ein Stimulator oder ein Monitoring Implantat mit einem gewebekontaktierenden Elektrodenpol oder einem Anschluss für einen einem gewebekontaktierenden Elektrodenpol ist, ist der oder einer der Systemintegritäts-Parameter vorzugsweise ein einen Elektroden-Gewebe-Kontakt charakterisierender Impedanzwert. Diese bevorzugte Ausführungsvariante beruht auf der Erkenntnis, dass der Impedanzwert zwischen einem Elektrodenpol und angrenzendem Gewebe sich in Folge MRT-bedingter Erwärmung besonders verändern und kritische Werte annehmen kann. Weiterhin ist es bevorzugt, wenn die Steuereinheit ausgebildet ist, das implantierbare medizinische Gerät auf ein ein MRT-typisches Magnet- und/oder elektromagnetisches Wechselfeld und/oder mechanische Schwingungen anzeigendes Ausgangssignal der MRT-Erkennungseinheit hin in einen MRT-Betriebsmodus zu versetzen, wobei die Steuereinheit weiterhin dazu ausgebildet ist, während des MRT-Betriebsmodus wiederkehrend eine Erfassung eines einen Elektroden-Gewebe-Kontakt charakterisierender Impedanzwertes zu veranlassen und erfasste Impedanzwerte hinsichtlich einer auf eine lokale Ödembildung hinweisenden Impedanzwertänderung auszuwerten. Ein solches implantierbares medizinisches Gerät kann bereits während einer MRT-Prozedur möglicherweise kritische Veränderungen und ggf. Ödembildungen erkennen und ggf. geeignete Gegenmaßnahmen wie z.B. eine Gewebekühlung oder ein Beenden der MRT-Prozedur veranlassen. Letzteres kann beispielsweise dadurch geschehen, dass das implantierbare medizinische Gerät Steuersignale an einen Kernspintomografen aussendet oder für bedienpersonal eines Kemspintomografen wahrnehmbare Signale aussendet.

Weiterhin ist die Steuereinheit vorzugsweise ausgebildet, einen Wegfall eines ein MRTtypischen Magnet- und/oder elektromagnetischen Wechselfelds und/oder mechanischen Schwingungen anzeigenden Ausgangssignals der MRT-Erkennungseinheit zu erfassen und daraufhin den MRT-Betriebsmodus zu beenden.

Im Falle einer Steuereinheit, die dazu ausgebildet ist, auf eine lokale Ödembildung hinweisenden Impedanzwertänderungen zu erfassen, ist die Steuereinheit vorzugsweise weiterhin ausgebildet Ödem-mildernde Maßnahmen, insbesondere eine Gewebekühlung in Bereich eines betroffenen Elektrodenpols, auszulösen, sobald sie auf eine lokale Ödembildung hinweisenden Impedanzwertänderungen erfasst hat.

In jedem Fall ist es bevorzugt, wenn das implantierbare medizinische Gerät eine Telemetrieeinheit aufweist, die angeschlossen und ausgebildet ist, Werte von einem oder mehreren Systemintegrationsparametern an ein externes Gerät zu übertragen. Dann könne diese werte nämlich auch in einem zentralen Service-Center ausgewertet und/oder weiter an einen betreuenden Arzt übermittelt werden.

Vorzugsweise ist die Telemetrieeinheit mit der Steuereinheit verbunden und ausgebildet ist, Steuerbefehle von einem externen Gerät zu empfangen; so dass das implantierbare medizinische Gerät mittels Steuerbefehlen von außerhalb, ggf. auch ferngesteuert durch ein zentrales Service-Center, so gesteuert werden kann, wie durch die erfassten Werte der Systemintegritätsparameter angezeigt ist.

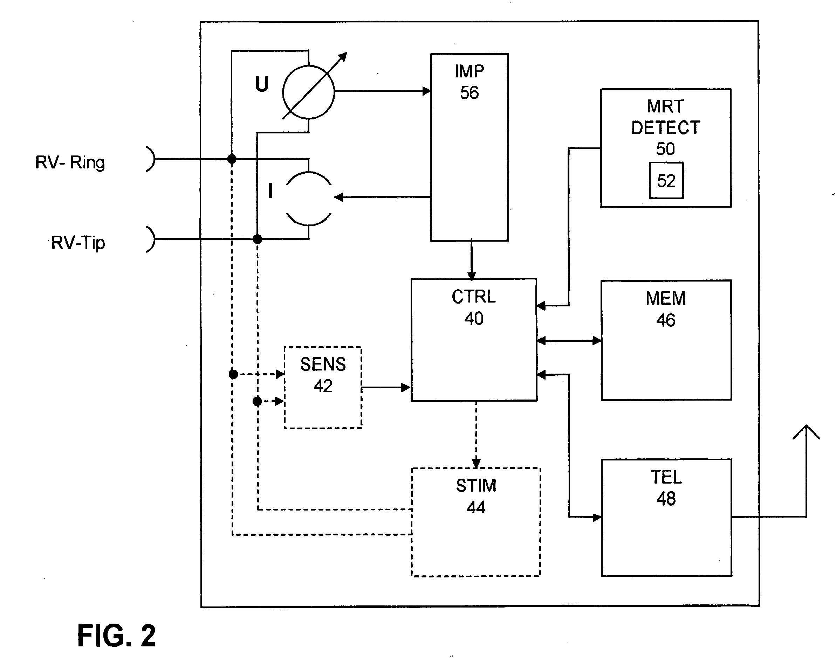

Die Testeinheit weist vorzugsweise eine Impedanzbestimmungseinheit auf oder ist mit einer solchen verbunden. Wie nachfolgend im Zusammenhang mit Figur 2 beschrieben können mit Hilfe einer solchen Impedanzbestimmungseinheit eine Reihe von Werten von Systemintegritätsparametem erfasst werden.

Falls das implantierbare elektromedizinische Gerät ein Herzstimulator ist, der ausgebildet ist Stimulationsimpulse abzugeben, die eine stimulierte Kontraktion einer Herzkammer bewirken können, ist die Testeinheit vorzugsweise dazu ausgebildet, eine automatische Stimulationserfolgskontrolle durchzuführen. Eine solche automatische Stimulationserfolgskontrolle ist auch als Automatic Capture Control (ACC) bekannt und kann auf vielfältige, an sich bekannte Weise, z.B. via Impedanzmessung, erfolgen.

Die Erfindung soll nun anhand von Ausführungsbeispielen mit Bezug auf die Figuren näher erläutert werden. Die Figuren zeigen Folgendes:
- FIG. 1: zeigt als implantierbare medizinische Geräte einen implantierbaren Herzstimulator 10 und eine daran angeschlossene implantierbare Elektrodenleitung 20.
- FIG. 2: zeigt beispielhaft einige Komponenten eines erfindungsgemäßen implantierbaren medizinischen Gerätes wie es beispielsweise in Figur 1 dargestellt ist.
- FIG. 3: erläutert den Ablauf einer MRT-Prozedur wie gemäß dem Stand der Technik nötig ist.
- FIG. 4: zeigt den Ablauf einer MRT-Prozedur mit einem erfindungsgemäßen Implantat.
- FIG. 5: zeigt, wie ein Implantat 10 über ein als körper-externe Relaisstation dienendes Patientengerät 300 telemetrisch an ein zentrales Service-Center 310 angebunden ist.

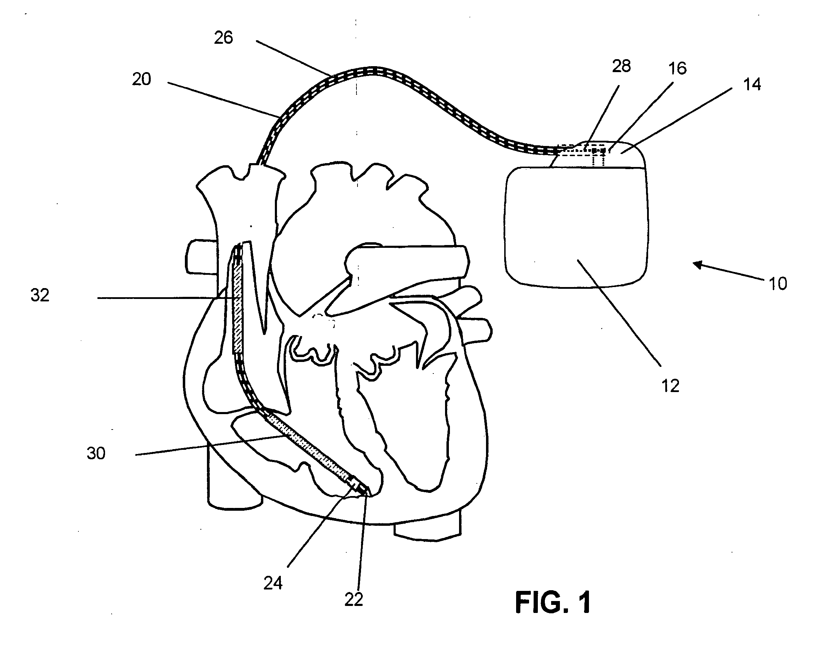

In Figur 1 ist ein implantierbares medizinisches Gerät in Form eines implantierbaren Herzstimulators 10 dargestellt, an den eine Elektrodenleitung 20, die einen langgestreckten Leiter aufweist, angeschlossen ist.

Der implantierbare Herzstimulator 10 kann ein Herzschrittmacher oder ein Kardioverter/Defibrillator (ICD) sein. In dem in Figur 1 dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 stellt im Sinne dieser Erfindung ein implantierbares medizinisches Gerät mit einem langgestrecktem, elektrischem Funktionsleiter dar. An einem distalen Ende der Elektrodenleitung 20 sind in an sich bekannter Manier Elektrodenpole in Form einer Spitzen- oder Tipelektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. FIG. 1 zeigt, wie sich die Elektrodenpole, also die Tipelektrode 22 und die Ringelektrode 24, im Anwendungsfall die Elektrodenleitung 20, im Apex eines rechten Ventrikels eines Herzens befinden.

Sowohl die Tipelektrode 22 als auch die Ringelektrode 24 sind über jeweils wenigstens einen elektrischen Leiter 26 mit einem Kontakt eines Steckers 28 am proximalen Ende der Elektrodenleitung 20 elektrisch verbunden. Der Stecker 28 besitzt elektrische Kontakte, die mit den elektrischen Kontakten 16 der Kontaktbuchse im Anschlussgehäuse 14 des implantierbaren Herzstimulators korrespondieren.

Die elektrischen Leiter 26 in der Elektrodenleitung 20 können als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden dienen dazu, elektrische Signale von Steckkontakt zum jeweiligen Elektrodenpol zu übertragen oder abgefühlte elektrische Potentiale repräsentierende Signale vom jeweiligen Elektrodenpol zum Steckkontakt zu führen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann aber jedes andere an sich bekannte elektromedizinische Implantat, also auch ein Mehrkammer-Herzschrittmacher oder Kardioverter/Defibrillator (ICD) oder auch ein Neurostimulator oder ein reines Monitoring-Implantat dienen.

Figur 2 zeigt beispielhaft und schematisch einige Komponenten des Herzstimulators 10 aus Figur 1. Typische Komponenten eines solchen Herzstimulators sind eine Steuereinheit 40, eine oder mehrere Sensingeinheiten 42, die jeweils eine Diagnoseeinheit darstellen und eine oder mehrere Stimulationseinheiten 44, die jeweils eine Therapieeinheit darstellen. Die Steuereinheit 40 ist zuvor mit der Sensingeinheit 42 als auch mit der Stimulationseinheit 44 verbunden. Sowohl die Sensingeinheit 42 als auch die Stimulationseinheit 44 sind jeweils mit Elektrodenanschlüssen verbunden, um im Falle der Sensingeinheit 42 elektrische Potentiale des Herzgewebes über die rechtsventrikuläre Ringelektrode 24 und/oder die rechtsventrikuläre Tipelektrode 22 erfassen zu können bzw. im Falle der Stimulationseinheit 44 Stimulationsimpulse beispielsweise über die rechtsventrikuläre Ringelektrode 24 und/oder die rechtsventrikuläre Tipelektrode 22 ausgeben zu können.

Außerdem ist die Steuereinheit 40 mit einer Speichereinheit 46 zum Speichern erfasster Werte von jeweils zu messenden Parametern verbunden. Eine ebenfalls mit der Steuereinheit 40 verbundene Telemetrieeinheit 48 erlaubt es, erfasste Werte von Parametern an ein externes Gerät zu übertragen oder Steuerbefehle von einem externen Gerät zu empfangen.

Die Steuereinheit 40 ist außerdem mit einer MRT-Erkennungseinheit 50 verbunden, die dazu ausgebildet ist, MRT-typische Magnet- und/oder elektromagnetisches Wechselfeld und/oder mechanische Schwingungen zu erkennen und ein Ausgangssignal an die Steuereinheit 40 auszugeben, welches das Vorhandensein solcher MRT-typischer Magnetund/oder elektromagnetisches Wechselfeld und/oder mechanische Schwingungen anzeigt. Die MRT-Erkennungseinheit 50 besitzt hierzu einen Magnetfeldsensor 52 und/oder Sensoren zur Detektion von elektromagnetischen Wechselfeldern und/oder Sensoren zur Detektion von mechanischen Schwingungen.

Die Steuereinheit 40 ist außerdem mit einer Testeinheit in Form einer Impedanzbestimmungseinheit 56 verbunden. Die Impedanzbestimmungseinheit 56 ist mit einer Stromquelle I und einer Spannungsmesseinheit U verbunden, die ihrerseits wiederum mit den Anschlüssen für die Ringelektrode 24 und die Tipelektrode 22 verbunden sind. Auf diese Weise kann die Gleichstromquelle I konstant Stromimpulse über die Tipelektrode 22 und die Ringelektrode 24 abgeben und die Spannungsmesseinheit U kann die dabei jeweils abfallende Spannung messen. Aus diesen Werten kann die Impedanzbestimmungseinheit 56 einen jeweiligen Impedanzwert bestimmen.

Ein so bestimmter Impedanzwert hängt von verschiedenen Einflussgrößen ab. Beispielsweise würde sich ein Bruch eines elektrischen Leiters in der Elektrodenleitung 20 in einem sehr hohen Impedanzwert äußern. Bei intakter Elektrodenleitung 20 hängt die zwischen den Elektrodenpolen 22 und 24 zu messende Impedanz auch von der Blutmenge im rechten Ventrikel eines Herzens ab, so dass die zu messende Impedanz entsprechend dem Herzzyklus zyklisch schwankt. Beispielsweise steigt die Impedanz mit abnehmendem Blutvolumen, d. h. mit abnehmendem Volumen der rechten Herzkammer, so dass ein zyklischer Anstieg der Impedanz die zyklische Kontraktion der rechten Herzkammer (des rechten Ventrikels) anzeigt. Ebenso kann ein entsprechender Anstieg der gemessenen Impedanz infolge einer Kammerkontraktion nach Abgabe eines Stimulationsimpulses den Stimulationserfolg anzeigen. Auf diese Weise ist die Impedanzbestimmungseinheit 56 in der Lage, eine automatische Stimulationserfolgskontrolle (automatic capture control; ACC) durchzuführen.

Die gemessene Impedanz hängt außerdem von der Impedanz des Elektrodenpol-Gewebekontaktes ab. Daher lässt sich durch Auswerten der gemessenen Impedanzwerte auch eine Ödembildung erfassen, wie sie beispielsweise durch eine Erwärmung der Elektrodenpole infolge magnetischer Wechselfelder eines Kemspintomografen auftreten kann.

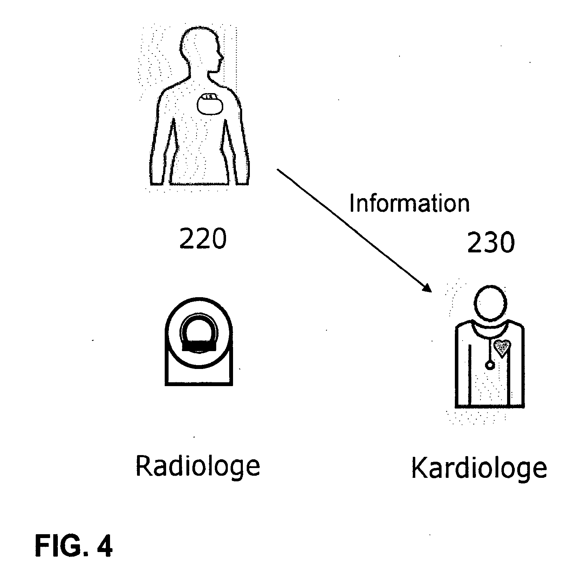

In der Figur 4 ist die gewünschte MRT-Prozedur für einen Patienten mit elektronischem Implantat und damit die Aufgabenstellung dargestellt. Beim Patienten kann ohne vorherige Nachsorge beim Implantatspezialisten die MRT-Untersuchung (220) durchgeführt werden und der Implantatspezialist (230) wird automatisch über die stattgefundene Untersuchung informiert und erhält dabei einen Nachweis der Systemintegrität, ohne den Patienten direkt untersuchen zu müssen. Die Systemintegritätsprüfung soll dabei gegenüber Figur 3 zusätzlich durch weitere automatische Verlaufsuntersuchungen verbessert werden.

Die Funktionsweise des Herzstimulators 10 oder eines anderen, Aspekte der hier beschriebenen Erfindung verwirklichenden elektronischen Implantats ist vorzugsweise wie folgt:

Wird vom elektronischen Implantat eine MRT-Umgebung automatisch erkannt (mit z.B. einem Sensor wie in z.B. in der parallel anhängigen Patentanmeldung US 12/970,290 beschrieben ist), werden dadurch getriggert mit einer vorgegebenen Verzögerung automatische Vor-Tests zum Erfassen bestimmter Parameter so z.B. Reizschwelle, Elektrodenimpedanz, Batteriespannung, Signalamplituden etc. ausgelöst. Danach wird der behandelnde Implantatspezialist über ein zentrales Service-Center automatisch darüber informiert, dass sein Patient eine MRT-Untersuchung hatte und erhält so per Datenfernübertragung die Daten - also die erfassten Werte der Systemintegritätsparameter - die er nach heutigem Stand der Technik selbst hätte erheben müssen.

Wird von der MRT-Erkennungseinheit 50 ein MRT erkannt, startet die Steuereinheit 40 unmittelbar einen "Vorabcheck des Patienten" wie nach heutigen Richtlinien vor einer MRT Untersuchung üblich. Im Rahmen eines solchen Vor-Tests werden z.B. Elektrodendaten wie Impedanz, Reizschwelle, Signalamplituden etc. automatisch vom Implantat bestimmt und bewertet. Wird dieser Test nicht bestanden macht sich das Implantat dem MRT Gerät - also dem Kemspintomografen - gegenüber mit Mitteln bemerkbar wie sie z.B. in der parallel anhängigen Patentanmeldung US 12/972,452 offenbart sind und die MRT-Prozedur wird abgebrochen.

Erfasst die MRT-Erkennungseinheit 50 ein MRT-typisches Magnet- und/oder elektromagnetisches Wechselfeld und/oder mechanische Schwingungen, löst die Steuereinheit 40 unmittelbar einen MRT-Betriebsmodus aus und damit eine Impedanzüberwachung zur Charakterisierung des Elektrode-Gewebe-Kontakts um eine eventuelle lokale Ödembildung festzustellen, die beispielsweise Folge zu hoher Erwärmung oder sonstiger Reizung (durch Vibration etc.) des Gewebes sein kann. Die Steuereinheit beendet den MRT-Betriebsmodus und damit die Impedanzüberwachung nach "MRI Ende erkannt" und sendet die Ergebnisse mittels der Telemetrieeinheit 48 an ein zentrales Service-Center. Wird ein Schwellwert für die erfasste Impedanz überschritten, wird dies als Hinweis auf ein Ödem gewertet. Dies veranlasst die Steuereinheit zum Auslösen eines Signals an den Kemspintomografen und/oder die Steuereinheit löst mildernde Maßnahmen aus, ggf. unter Inkaufnahme höheren Energieverbrauchs. Solche mildernde Maßnahmen können z.B. aktive Verfahren wie z.B. eine Kühlung mittels eines Peltier-Elements (das dann ebenfalls Bestandteil des medizinischen Gerätes wäre) sein. Die Aufzeichnung erfassten Werte der Systemintegrationsparameter läuft auch in den Holter um später auslesbar zu sein oder für den Fall dass keine Funkverbindung für eine drahtlose Telemetrie über die Telemetrieeinheit besteht oder für den jeweiligen Patienten keine Anbindung an eine zentrales Service-Center besteht.

Eine Impedanzmessung im Rahmen der Bestimmung von Werten der Systemintegritätsparameter erfolgt in einem Frequenzbereich von mehr als 100 kHz ausgenommen die Bänder +/- 5 MHz um die für den jeweiligen MR Scanner typische Larmor-Frequenz. Dadurch wird eine gegenseitige Störung vermieden, d.h. die Impedanzmesssgung des Implantats wird nicht durch die Scan-Sequenz gestört und umgekehrt die Impedanzmesssgung des Implantats stört nicht die Bildgebung. Um durch die erfindungsgemäße Lösung verschiedenen gängigen MR-Typen Rechung zu tragen (z.B. 1.5T, 3T, 7T MRT) werden Impedanzmessungen in den Frequenzbereichen:100kHz - 59MHz, 69MHz - 123MHz, 133MHz - 290MHz, >300 MHz bevorzugt. Andere während einer MRT-Prozedur kontinuierlich oder sporadisch zu überwachende Parameter sind z.B. eine Überprüfung der Reizschwelle des Herzgewebes. Die Reizschwelle des Herzgewebes gibt an, welche Stärke ein Stimulationsimpuls überschreiten muss, damit er eine stimulierte Kontraktion einer jeweiligen Herzkammer bewirkt. Im Rahmen eines Reizschwellentests werden typischerweise Stimulationsimpulse unterschiedlicher Stärke an das Herzgewebe angeben und anschließend jeweils eine Stimulationserfolgskontrolle durchgeführt. So lässt sich ermitteln, ab welcher Stimulationsimpulsstärke ein Stimulationserfolg eintritt und wo mithin die Reizschwelle des jeweiligen Herzgewebes liegt.

Nach von der MRT-Erkennungseinheit erfasstem Abschluss einer MRT-Untersuchung führt das Implantat in einem Post-MRT-Betriebsmodus zyklisch eine Verlaufskontrolle der Systemintegritätsparameter durch und vergleicht diese jeweils mit der Parametern von der MRT-Untersuchung und informiert bei einer Parameterdrift nach der MRT-Untersuchung den Implantatspezialisten per telemetrischer Datenübertragung an das zentrale Service-Center.

Nach von der MRT-Erkennungseinheit erfasstem Abschluss einer MRT-Untersuchung startet versetzt die Steuereinheit 40 das Implantat in einen "Automatischen Post MRT-Modus mit automatischer Datenübertragung", wobei die im Post MRT-Modus zu übertragenden Werte durch den Parametervergleich der Messwerte vor und nach der MRT-Untersuchung bestimmt werden. Beendet wird der Post MRT-Modus entweder, wenn die Steuereinheit 40 und/oder das zentrale Service eine "Normalisierung" der gemessenen Werte der Systemintegritätsparameter feststellen, die weiter im Verlauf automatisch vom Implantat überwacht werden oder aber über ein Fernprogrammierkommando, das vom Implantatspezialisten über das zentrale Service-Center ausgelöst wird.

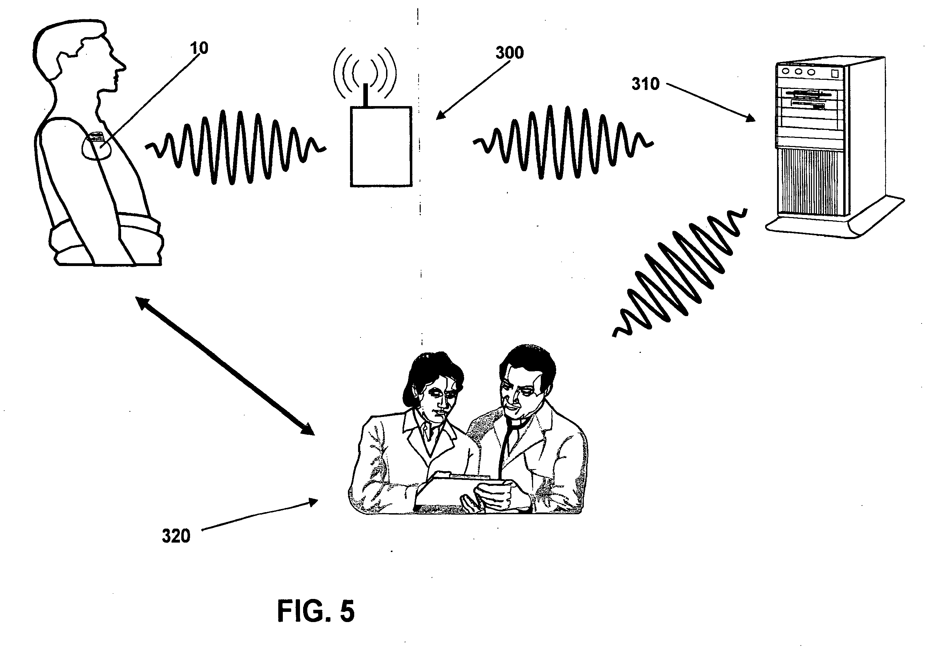

Figur 5 zeigt, wie ein Implantat 10 über ein als körper-externe Relaisstation dienendes Patientengerät 300 telemetrisch an ein zentrales Service-Center 310 angebunden ist. In dem Service-Center 310 können seitens des Implantats 10 empfangene Werte ausgewertet und bewertet werden. Die hierzu zur Verfügung stehende Rechenkapazität ist im Service-Center höher als im Implantat. Außerdem können im Service-Center Werte eines Implantats mit Werten anderer, ähnlicher oder identischer Implantate verglichen werden.

Über das zentrale Service-Center können jeweils auch ein betreuender Arzt (Kardiologe) oder ein Implantatsspezialist 320 informiert werden, die ggf. via zentrales Service-Center oder direkt auf das Implantat 10 einwirken können, falls beispielsweise infolge einer MRT-Einwirkung verschlechterter Werte von Systemintegritätsparametern entsprechender Handlungsbedarf besteht.

Das hier für MRT-typische und ähnliche Magnet- und/oder elektromagnetisches Wechselfeld und/oder mechanische Schwingungen beschriebene Konzept kann auch auf andere derzeit kontraindizierter Therapie- und Diagnoseverfahren, wie z.B. Therapeutische Bestrahlung etc. übertragen werden. In diesem Fall ist die MRT-Erkennungseinheit durch eine andere, aus das jeweilige Diagnose- oder Therapieverfahren abgestellte Therapieerkennungseinheit zu ersetzen.

Darüber hinaus werden auch Wechselfelder mechanischer Natur wie Vibrationen oder Akustische Signale verwendet, anhand derer das Vorhandensein oder nicht mehr Vorhandensein eines Störfeldes erkannt wird, um daran orientiert die beschriebenen Funktions-Modi des Implantats zu schalten.

Die erfindungsgemäße Lösung bietet den Vorteil, das eine erhebliche Vereinfachung einer MRT-Prozedur für Patienten mit elektronische Implantaten möglich wird, ohne die Patientensicherheit gegenüber dem derzeit zugelassenen Prozess einzuschränken.

## Patentansprüche

1. Implantierbares elektromedizinisches Gerät mit
- einer Erkennungseinheit zum Erfassen ggf. gerätebeeinträchtigender Einwirkungen
- einer Steuereinheit, die mit der Erkennungseinheit verbunden ist
- einer Diagnose-Therapieeinheit mit einer Diagnose- und/oder Therapieeinheit und mit
- einer Testeinheit
von denen die Testeinheit ausgebildet ist, die Diagnose-Therapieeinheit oder Komponenten der Diagnose-Therapieeinheit zu testen und so gewonnene Testergebnisse zum Speichern auszugeben,
von denen die Diagnose-Therapieeinheit Sensoreinheiten und/oder Therapieabgabeeinheiten als Komponenten aufweist und ausgebildet ist physiologische Parameter aufzunehmen und/oder eine Therapieabgabe zu bewirken, und
von denen die Steuereinheit ausgebildet ist, die Testeinheit zum Testen der Diagnose-Therapieeinheit anzusteuern,
wobei die Steuereinheit ausgebildet ist, auf ein ggf. gerätebeeinträchtigende Einwirkungen anzeigendes Ausgangssignal der Erkennungseinheit hin einen Vor-Test wenigstens einer Komponente der Diagnose-Therapieeinheit durch die Testeinheit auszulösen, wobei die Testeinheit während des Vor-Tests Werte von Systemintegritäts-Parametem erfasst, die eine Betriebsfähigkeit des implantierbaren medizinischen Gerätes charakterisieren, **dadurch gekennzeichnet, dass** das implantierbare elektromedizinische Gerät ein Stimulator oder ein Monitoring Implantat mit einem gewebekontaktierenden Elektrodenpol oder einem Anschluss für einen gewebekontaktierenden Elektrodenpol ist und ein Systemintegritäts-Parameter ein einen Elektroden-Gewebe-Kontakt charakterisierender Impedanzwert ist.

2. Implantierbares elektromedizinisches Gerät nach Anspruch 1, **dadurch gekennzeichnet, dass** die Erkennungseinheit eine MRT-Erkennungseinheit zum Erfassen MRT-typischer Magnetfelder und/oder elektromagnetischer Wechselfelder und/oder mechanischer Schwingungen

3. Implantierbares elektromedizinisches Gerät nach Anspruch 2, **dadurch gekennzeichnet, dass** die Steuereinheit ausgebildet ist, einen Wegfall MRT-typischer Magnetfelder und/oder elektromagnetischer Wechselfelder und/oder mechanischer Schwingungen anzeigenden Ausgangssignals der MRT-Erkennungseinheit zu erfassen und daraufhin einen Nach-Test durch die Testeinheit auszulösen.

4. Implantierbares elektromedizinisches Gerät nach Anspruch 3, **dadurch gekennzeichnet, dass** die Steuereinheit ausgebildet ist, das implantierbare medizinische Gerät im Anschluss an einen Nach-Test in einen Post-MRT-Betriebsmodus zu versetzen, in dem zyklisch Nach-Tests erfolgen, wobei die Steuereinheit weiterhin dazu ausgebildet ist, Werte eines jeweiligen Systemintegritäts-Parameters aus einem jeweiligen Nach-Test mit einem entsprechenden Wert desselben Systemintegritäts-Parameters aus den letzten Vor-Test zu vergleichen und den Post-MRT-Betriebsmodus zu beenden, wenn eine Abweichung der Werte unterhalb eines vorgegeben Schwellwertes liegt.

5. Implantierbares elektromedizinisches Gerät nach einem der Ansprüche 1 bis 4, **dadurch gekennzeichnet, dass** die Steuereinheit ausgebildet ist, das implantierbare medizinische Gerät auf MRT-typische Magnetfelder und/oder elektromagnetischer Wechselfelder und/oder mechanischer Schwingungen anzeigendes Ausgangssignal der MRT-Erkennungseinheit hin in einen MRT-Betriebsmodus zu versetzen, wobei die Steuereinheit weiterhin dazu ausgebildet ist, während des MRT-Betriebsmodus wiederkehrend eine Erfassung eines einen Elektroden-Gewebe-Kontakt charakterisierender Impedanzwertes zu veranlassen und erfasste Impedanzwerte im Hinblick auf möglicherweise kritische Impedanzwertänderungen auszuwerten.

6. Implantierbares elektromedizinisches Gerät nach einem der Ansprüche 1 bis 5, **dadurch gekennzeichnet, dass** die Steuereinheit ausgebildet ist, einen Wegfall MRT-typischer Magnetfelder und/oder elektromagnetischer Wechselfelder und/oder mechanischer Schwingungen anzeigenden Ausgangssignals der MRT-Erkennungseinheit zu erfassen und daraufhin den MRT-Betriebsmodus zu beenden.

7. Implantierbares elektromedizinisches Gerät nach einem der Ansprüche 1 bis 6, **dadurch gekennzeichnet, dass** die Steuereinheit ausgebildet ist, auf eine lokale Ödembildung hinweisende Impedanzwertänderungen zu erfassen und daraufhin Ödemmildernde Maßnahmen auszulösen.

8. Implantierbares elektromedizinisches Gerät nach einem der Ansprüche 1 bis 7, **dadurch gekennzeichnet, dass** das implantierbare medizinische Gerät eine Telemetrieeinheit aufweist, die angeschlossen und ausgebildet ist, Werte von einem oder mehreren Systemintegrationsparametem an ein externes Gerät zu übertragen.

9. Implantierbares elektromedizinisches Gerät nach Anspruch 8, **dadurch gekennzeichnet, dass** die Telemetrieeinheit mit der Steuereinheit verbunden und ausgebildet ist, Steuerbefehle von einem externen Gerät zu empfangen.

10. Implantierbares elektromedizinisches Gerät nach einem der Ansprüche 1 bis 9, **dadurch gekennzeichnet, dass** die Testeinheit eine Impedanzbestimmungseinheit ausweist oder mit einer solchen verbunden ist.

11. Implantierbares elektromedizinisches Gerät nach Anspruch 10, **dadurch gekennzeichnet, dass** die Impedanzbestimmungseinheit ausgebildet ist, eine jeweilige Impedanzmessung in einem Frequenzbereich größer 100kHz und ausgenommen die Frequenzbänder +/- 5MHz um die MR-typischen Larmor-Frequenzen durchzuführen.

12. Implantierbares elektromedizinisches Gerät nach einem der Ansprüche 1 bis 11, **dadurch gekennzeichnet, dass** das implantierbare elektromedizinische Gerät ein Herzstimulator ist, der ausgebildet ist Stimulationsimpulse abzugeben, die eine stimulierte Kontraktion einer Herzkammer bewirken können, die Testeinheit ausgebildet ist, eine automatische Stimulationserfolgskontrolle durchzuführen.

## Claims

1. An implantable electromedical device, comprising
- a detection unit for capturing effects potentially detrimental to the device,
- a control unit, which is connected to the detection unit,
- a diagnosis/therapy unit having a diagnosis and/or therapy unit, and comprising
- a test unit,
of which the test unit is designed to test the diagnosis/therapy unit or components of the diagnosis/therapy unit and to output test results thus obtained for storage purposes, of which the diagnosis/therapy unit comprises sensor units and/or therapy administration units as components and is designed to record physiological parameters and/or to administer a therapy, and
of which the control unit is designed to activate the test unit to test the diagnosis/therapy unit,
wherein the control unit, in response to an output signal of the detection unit indicating effects potentially detrimental to the device, is designed to trigger a pre-test of at least one of the components of the diagnosis/therapy unit by means of the test unit, wherein the test unit during the pre-test detects system integrity parameters characterising an operating ability of the implantable medical device, **characterised in that** the implantable electromedical device is a stimulator or a monitoring implant with an electrode pole contacting tissue, and a system integrity parameter is an impedance value characterising electrode/tissue contact.

2. The implantable electromedical device according to Claim 1, **characterised in that** the detection unit is an MRT detection unit for capturing MRT-typical magnetic fields and/or electromagnetic alternating fields and/or mechanical vibrations.

3. The implantable electromedical device according to Claim 2, **characterised in that** the control unit is designed to capture a disappearance of an output signal of the MRT detection unit indicating MRT-typical magnetic fields and/or electromagnetic alternating fields and/or mechanical vibrations and, in response, to trigger a post-test by means of the test unit.

4. The implantable electromedical device according to Claim 3, **characterised in that** the control unit, after a post-test, is designed to shift the implantable medical device into a post-MRT operating mode, in which post-tests are carried out cyclically, wherein the control unit is furthermore designed to compare values of a respective system integrity parameter from a respective post-test with a corresponding value of the same system integrity parameter from the last pre-test and to end the post-MRT operating mode when a deviation between the values lies below a predefined threshold value.

5. The implantable electromedical device according to one of Claims 1 to 4, **characterised in that** the control unit is designed to shift the implantable medical device into an MRT operating mode in response to an output signal of the MRT detection unit indicating MRT-typical magnetic fields and/or electromagnetic alternating fields and/or mechanical vibrations, wherein the control unit is furthermore designed, during the MRT operating mode, to periodically prompt a capturing of an impedance value characterising electrode/tissue contact and to evaluate captured impedance values in view of potential critical impedance value changes.

6. The implantable electromedical device according to one of Claims 1 to 5, **characterised in that** the control unit is designed to capture a disappearance of an output signal of the MRT detection unit indicating MRT-typical magnetic fields and/or electromagnetic alternating field and/or mechanical vibrations and, in response, to end the MRT operating mode.

7. The implantable electromedical device according to one of Claims 1 to 6, **characterised in that** the control unit is designed to capture impedance value changes indicating a local oedema formation and to trigger oedema-alleviating measures in response.

8. The implantable electromedical device according to one of Claims 1 to 7, **characterised in that** the implantable medical device comprises a telemetry unit, which is connected and designed to transfer values from one or more system integration parameters to an external device.

9. The implantable electromedical device according to Claim 8, **characterised in that** the telemetry unit is connected to the control unit and is designed to receive control commands from an external device.

10. The implantable electromedical device according to one of Claims 1 to 9, **characterised in that** the test unit comprises an impedance determination unit or is connected to such a unit.

11. The implantable electromedical device according to Claims 10, **characterised in that** the impedance determination unit is designed to carry out a respective impedance measurement in a frequency range greater than 100 kHz, apart from the frequency bands +/- 5 MHz around the MR-typical Larmor frequencies.

12. The implantable electromedical device according to one of Claims 1 to 11, **characterised in that** the implantable electromedical device is a cardiac stimulator, which is designed to output stimulation pulses that can cause a stimulated contraction of a heart ventricle, and the test unit is designed to carry out an automatic stimulation success check.

## Revendications

1. Dispositif électro-médical implantable, avec
- une unité de reconnaissance pour la détection d'effets nuisible au dispositif,
- une unité de commande reliée à l'unité de reconnaissance,
- une unité de diagnostic thérapeutique avec une unité de diagnostic et/ou de thérapie, et avec
- une unité de test
parmi lesquelles l'unité de test est conçue pour tester l'unité de diagnostic thérapeutique ou des composants de l'unité de diagnostic thérapeutique et délivrer les résultats de test ainsi obtenus pour l'enregistrement,
parmi lesquelles l'unité de diagnostic thérapeutique comporte des unité de capteurs et/ou des unités de délivrance de traitement en tant que composants, tout en étant conçue pour enregistrer des paramètres physiologiques et/ou provoquer la délivrance d'un traitement, et
parmi lesquelles l'unité de commande est conçue pour commander l'unité de test pour tester l'unité de diagnostic thérapeutique,
dans lequel l'unité de commande est conçue pour déclencher un pré-test d'au moins un composant de l'unité de diagnostic thérapeutique par l'unité de test, suite à un signal de sortie de l'unité de reconnaissance indiquant des effets nuisibles au dispositif, dans lequel l'unité de test collecte des valeurs de paramètres d'intégrité du système pendant le pré-test, lesquels caractérisent un état opérationnel du dispositif médical implantable, **caractérisé en ce que** le dispositif électro-médical implantable est un stimulateur ou un implant de surveillance, avec un pôle d'électrode en contact avec les tissus ou un raccordement pour un pôle d'électrode en contact avec les tissus, et **en ce qu'**un paramètre d'intégrité du système est une valeur d'impédance caractérisant un contact entre les tissus et l'électrode.

2. Dispositif électro-médical implantable selon la revendication 1, **caractérisé en ce que** l'unité de reconnaissance est une unité de reconnaissance par IRM pour la détection de champs magnétiques d'IRM typiques et/ou de champs électromagnétiques alternatifs et/ou d'oscillations mécaniques.

3. Dispositif électro-médical implantable selon la revendication 2, **caractérisé en ce que** l'unité de commande est conçue pour détecter une disparition d'un signal de sortie de l'unité de reconnaissance par IRM, indiquant de champs magnétiques d'IRM typiques et/ou de champs électromagnétiques alternatif et/ou des oscillations mécaniques, puis de déclencher un post-test par l'unité de test.

4. Dispositif électro-médical implantable selon la revendication 3, **caractérisé en ce que** l'unité de commande est conçue pour faire passer le dispositif médical implantable dans un mode de fonctionnement post-IRM suite à un post-test, dans lequel des post-tests sont effectués cycliquement, l'unité de commande étant en outre conçue pour comparer des valeurs d'un des paramètres d'intégrité du système issues d'un des post-tests avec une valeur correspondante du même paramètre d'intégrité du système, issue du dernier pré-test, et pour mettre fin au mode de fonctionnement post-IRM lorsqu'un écart des valeurs est inférieur à une valeur seuil prédéfinie.

5. Dispositif électro-médical implantable selon l'une des revendications 1 à 4, **caractérisé en ce que** l'unité de commande est conçue pour faire passer le dispositif médical implantable dans un mode de fonctionnement d'IRM suite à un signal de sortie de l'unité de reconnaissance par IRM, indiquant de champs magnétiques d'IRM typiques et/ou de champs électromagnétiques alternatifs et/ou des oscillations mécaniques, dans lequel l'unité de commande est en outre conçue pour déclencher durant le mode de fonctionnement IRM une détection périodique d'une valeur d'impédance caractérisant un contact entre l'électrode et les tissus, et pour évaluer les valeurs d'impédance détectées, quant à d'éventuelles modifications critiques de la valeur d'impédance.

6. Dispositif électro-médical implantable selon l'une des revendications 1 à 5, **caractérisé en ce que** l'unité de commande est conçue pour détecter une disparition d'un signal de sortie de l'unité de reconnaissance par IRM, indiquant de champs magnétiques d'IRM typiques et/ou de champs électromagnétiques alternatifs et/ou des oscillations mécaniques, et pour ensuite mettre fin au mode de fonctionnement d'IRM.

7. Dispositif électro-médical implantable selon l'une des revendications 1 à 6, **caractérisé en ce que** l'unité de commande est conçue pour détecter des modifications de valeur d'impédance indiquant un oedème local, et pour ensuite déclencher des mesures d'atténuation d'oedème.

8. Dispositif électro-médical implantable selon l'une des revendications 1 à 7, **caractérisé en ce que** le dispositif médical implantable comporte une unité de télémétrie raccordée et conçue pour transmettre des valeurs d'un ou de plusieurs paramètres d'intégrité du système à un dispositif externe.

9. Dispositif électro-médical implantable selon la revendication 8, **caractérisé en ce que** l'unité de télémétrie est reliée à l'unité de commande et conçue pour recevoir des ordres de commande venant d'un dispositif externe.

10. Dispositif électro-médical implantable selon l'une des revendications 1 à 9, **caractérisé en ce que** l'unité de test comporte une unité de détermination d'impédance ou est reliée à une telle unité.

11. Dispositif électro-médical implantable selon la revendication 10, **caractérisé en ce que** l'unité de détermination d'impédance est conçue pour effectuer une mesure d'impédance dans une plage de fréquences supérieure à 100kHz, à l'exception des bandes de fréquence +/- 5MHz autour des fréquences de Larmor typiques de la RM.

12. Dispositif électro-médical implantable selon l'une des revendications 1 à 11, **caractérisé en ce que** le dispositif médical implantable est un stimulateur cardiaque conçu pour délivrer des impulsions de stimulation capables de provoquer une contraction stimulée d'un ventricule, l'unité de test étant conçue pour effectuer un contrôle de stimulation automatique.
